# EUROPEAN PATENT APPLICATION

(11) **EP 0 855 175 A1**
(43) Date of publication of application: **29.07.1998**
(21) Application number: 98300432.6
(22) Date of filing: 21.01.1998
(51) Int. Cl.: A61F 5/08

(54) **Improvements relating to nasal dilators**

(30) Priority: 25.01.1997 GB 9701556
(71) Applicant: Innovative Technologies Limited, Tarvin, Cheshire CH3 8JF (GB)
(72) Inventor: Ives, Christopher L., Nantwich, Cheshire, CW5 8QA (GB); Harrison, Ian M., Knutsford, Cheshire, WA16 6LP (GB)
(74) Representative: McNeight, David Leslie

(57) **Abstract**

There is disclosed a nasal dilator comprising a unitary truss member having a normally substantially arcuate configuration, the truss member having:
a flexible member (14) adapted to locate on the bridge of the nose and on the outer wall tissue of the first and second nasal passages;
engagement means (20) secured to the convex surface of the flexible member, the engagement means being adapted to engage at least the outer wall tissue of the first and second nasal passages;
at least one resilient member (22) secured to the concave surface of the flexible member, the resilient member or member being sufficiently deformable to allow the truss member to engage the outer wall tissue of the first and second nasal passages and providing restoring forces when so engaged, said restoring forces acting to dilate the nasal passages.

## Description

This invention relates to the field of nasal dilators.

Devices which attach to the outer wall tissues of the human nose and which act to prevent drawing in of said outer wall tissues during breathing are well known. Such devices are primarily intended to alleviate blockage of the nasal passages, with particular attention being paid to the prevention of snoring. US Patents US 1 292 083 and US 1 950 839 are early examples of nasal dilators in which a pair of adhesive pads or suction pads are attached to the outer wall tissue of both nostrils, the pads being connected by a metal wire. The spring like action of the metal wire serves to separate outer wall tissue from inner wall tissue thereby dilating the nostrils.

More recently, International Publication WO 92/22340 has disclosed dilators which essentially comprise a flexible layer having adhesive on one major surface thereof and resilient member or members on the other major surface thereof. The adhesive secures the dilator to the outer wall tissues of the nostrils and the resilient member or members provide a restoring force when the device is flexed away from its normal, planar configuration. In use, the device is bent around the bridge of the nose, and the restoring forces act to dilate the nostrils.

However, WO 92/22340 is only concerned with preventing outer wall tissue of nasal passages of a nose from drawing in during breathing, as opposed to active dilation of the nostrils. Indeed, WO 92/22340 is primarily concerned with the alleviation of nasal blockage at night time which; *inter alia,* reduces the tendency to snore. In recent years, however, devices of the type described in WO 92/22340 have increasingly been employed by sportsmen and women. The rationale for this use is that dilation of the nostril increases air flow during breathing which in turn leads to increased oxygen uptake. Thus there is a need for nostril dilators which provide improved dilation, in particular for dilators that can provide "active" dilation, i.e. an increase in the cross sectional area of the nasal passages before inhalation commences.

Furthermore, there are problems associated with comfort for the wearer of the nostril dilator, and with a tendency of the device to peel away from the surface of the nose. These problems are in large measure associated with the distribution of forces over the device. International Publication WO 96/01093 discloses improvements to the design of WO 92/22340 which reduce the tendency of the dilator to peel away from the nose. The improvements essentially consist of the provision of a series of notches in the resilient member or members which reduce the spring constant of the member(s) at the ends thereof.

The present invention addresses the above mentioned problems and provides a convenient, comfortable and skin friendly device capable of powerful nostril dilation. These advantages are provided by careful selection of the configuration of the dilator and of the materials employed therein.

According to the invention there is provided a nasal dilator comprising a unitary truss member having a normally substantially arcuate configuration, the truss member having:
a flexible member adapted to locate on the bridge of the nose and on the outer wall tissue of the first and second nasal passages;
engagement means secured to the convex surface of the flexible member, the engagement means being adapted to engage at least the outer wall tissues of the first and second nasal passages;
at least one resilient member secured to the concave surface of the flexible member, the resilient member or members being sufficiently deformable to allow the truss member to engage the outer wall tissue of the first and second nasal passages and providing restoring devices when so engaged, said restoring forces acting to dilate the nasal passages.

The resilient member or members may comprise a thermo plastic . The resilient member or members may be secured to the flexible member before thermoforming.

The flexible member may comprise a polyurethane membrane.

The resilient member or members may be chemically bonded to the flexible member. The resilient member or members may be covalently bonded by a layer of cyanoacrylate.

The engagement means may comprise at least one layer of adhesive.

The unitary truss member may have a normally substantially arcurate configuration substantially described by a radius of curvature of less than 6 cm, preferably less than 4 cm.

The resilient member or members may comprise a thermo plastic selected from the group comprising: polyethyleneterephthalate; PVC and polyethylene.

The nasal dilator may further comprise first and second release liners covering the layer or layers of adhesive.

Nasal dilators according to the invention will now be described with reference to the accompanying drawings, in which:-
- Figure 1: shows a plan view of a first truss;
- Figure 2: shows a cross sectional view of the first truss along the line A-A¹ of Figure 1;
- Figure 3: shows (a) a view from below prior to application of the first truss and (b) after application of the first truss;
- Figure 4: shows plan views of a second and a third truss; and
- Figure 5: shows a plan view of a flexible member of alternative shape.

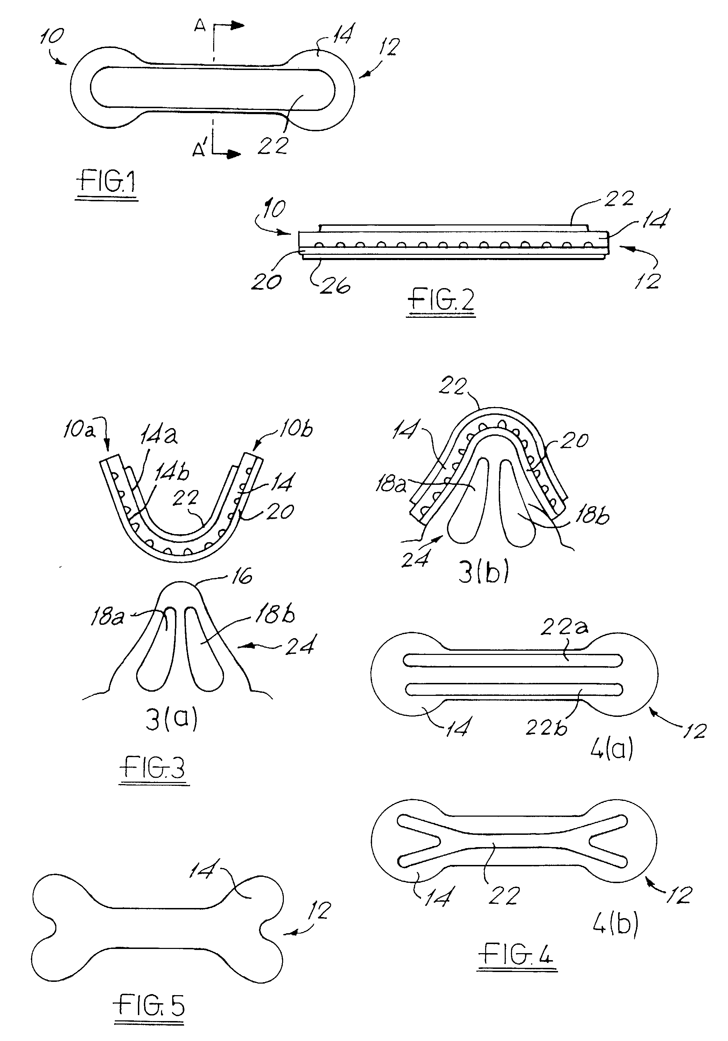

Figures 1 to 3 are various views of a nasal dilator 10 comprising a unitary truss member 12 having a normally substantially arcuate configuration (see Figure 3a), the truss member 12 having:
a flexible member 14 adapted to locate on the bridge 16 of the nose and on the outer wall tissue of the first 18a and second 18b nasal passages;
engagement means 20 secured to the convex surface 14a of the flexible member 14, the engagement means 20 being adapted to engage at least the outer wall tissue of the first 18a and second 18b nasal passages;
at least one resilient member 22 secured to the concave surface 14a of the flexible member 14, the resilient member 22 being sufficiently deformable to allow the truss member 12 to engage the outer wall tissue of the first 18a and second 18b nasal passages and providing restoring forces when so engaged, said restoring forces acting to dilate the nasal passages.

The resilient member 22 comprises a thermo plastic. Advantageously the resilient member 22 is first secured to the flexible member 14 before thermoforming.

In use, the nasal dilator 10 is positioned adjacent to a nose 24 in the manner shown in Figure 3a. The middle portion of the dilator 10 is then located on the bridge of the nose, and the ends of the dilator 10a, 10b are bent downwards to engage the outer wall tissue of the first 18a and second 18b nasal passages (see Figure 3b). Thus positioned the nasal dilator 10 of the present invention attempts to revert to its original, substantially arcuate geometry. The associated restoring forces exerted thereby are substantially greater than those resulting in a similar device of normally planar configuration, and hence the present invention results in improved nostril dilation.

Preferably, the flexible member 14 is a polyurethane membrane. There are a number of advantages associated with the use of such a membrane. The membrane provides a biocompatible, skin friendly substrate that absorbs relative stress between the hard, resilient member 22 and the skin (except, of course, the purely restoring force exerted by the resilient member 22). The good mechanical properties of the membrane permit compression, tension and some lateral, and "up" and "down" forces to be absorbed between the skin and the device without detachment thereof. This substantially reduces itchiness felt on the skin of the nose than can occur with devices that are too rigid. Furthermore, since uncomfortable, itchy sensations are reduced, the likelihood of detachment of the device by scratching or wrinkling of the bridge of the nose is much reduced. It is also convenient in this regard that the arcuate configuration of the dilator 10 optimises the restoring forces provided by a resilient member relative to the width and thickness thereof. By reducing the width of the resilient member whilst retaining useful restoring forces, the area of the dilator in contact with the bridge of the nose can be reduced, leading to increased comfort for the wearer.

An example of suitable polyurethane membrane dimensions are 0.4 mm thickness, 65 mm length and 22 mm maximum width, although the invention is in no way limited in this regard. The "dog bone" configuration illustrated in Figure 1 is useful since the surface area of the dilator 10 in contact with the bridge of the nose is minimised.

The resilient member 22 is chemically bonded to the polyurethane membrane 14. An example of a suitable bonding agent is cyanoacrylate, although other bonding agents would suggest themselves to one skillled in the art.

The engagement means 20 conveniently comprise a layer of adhesive. A single layer covering the whole of the convex surface of the polyurethane membrane 14 has been found to be preferable, although it is possible to apply two layers of adhesive, one at each end of the membrane 14, with no layer of adhesive on the portion of the membrane 14 that locates over the bridge of the nose.

Preferably, in order to provide substantially improved restoring forces, the unitary truss member 12 has a normally substantially arcuate configuration substantially described by a radius of curvature of less than 6 cm, preferably less than 4 cm.

Examples of suitable thermoformable plastics for use as the resilient member 22 include polyethylenephthalate, PVC and polyethylene.

The nasal dilator 10 further comprises first 26 and second (not shown) release liners covering the layer of adhesive 20. The release liners are removed prior to application ofthe truss 12 to the nose.

Figure 4a, 4b and 5 show some possible variations in the configuration of the truss. Figure 4a shows an example in which two resilient members 22a and 22b are employed. Figure 4b depicts a truss 12 having a single resilient member 22 which possesses two "forks" at each end of the device. In both of these configurations the restoring force applied by the resilient member(s) 22, which causes dilation of the nostrils, is increased relative to the dimensions of the resilient member(s) 22. In particular, the extent of the resilient member(s) 22 in the central portion of the truss 12, which in use is attached to the bridge of the nose, is relatively small, resulting in enhanced user comfort and a reduced tendency to peel away from the bridge of the nose. Figure 5 shows a variation in the shape of the flexible member 14.

A further advantage of the substantially arcurate configuration is that the truss 12 is easily positioned on the bridge of the nose. Examples of the present invention can provide restoring forces of 30 to 60 grams *in situ.*

## Claims

1. A nasal dilator comprising a unitary truss member having a normally substantially arcuate configuration, the truss member having:
a flexible member adapted to locate on the bridge of the nose and on the outer wall tissue of the first and second nasal passages;
engagement means secured to the convex surface of the flexible member, the engagement means being adapted to engage at least the outer wall tissue of the first and second nasal passages;
at least one resilient member secured to the concave surface of the flexible member, the resilient member or member being sufficiently deformable to allow the truss member to engage the outer wall tissue of the first and second nasal passages and providing restoring forces when so engaged, said restoring forces acting to dilate the nasal passages.

2. A nasal dilator according to claim 1 in which the resilient member or members comprise a thermo plastic.

3. A nasal dilator according to claim 2 in which the resilient member or members are secured to the flexible member before thermoforming.

4. A nasal dilator according to any of claims 1 to 3 in which the flexible member comprises a polyurethane membrane.

5. A nasal dilator according to any of the previous claims in which the resilient member or members are chemically bonded to the flexible member.

6. A nasal dilator according to claim 5 in which the resilient member or members are chemically bonded by a layer of cyanoacrylate.

7. A nasal dilator according to any of the previous claims in which the engagement means comprise at least one layer of adhesive.

8. A nasal dilator according to any of the previous claims in which the unitary truss member has a normally substantially arcuate configuration substantially described by a radius of curvature of less than 6 cm, preferably less than 4 cm.

9. A nasal dilator according to any of claims 2 to 8 in which the resilient member or members comprise a thermoset plastic selected from the group comprising : polyethylenetherphthalate; PVC and polyethylene.

10. A nasal dilator according to any of the previous claims further comprising first and second release liners covering the layer or layers of adhesive.
